# EUROPEAN PATENT SPECIFICATION

(11) **EP 2 495 306 B1**
(45) Date of publication and mention of the grant of the patent: **09.08.2017**
(21) Application number: 11001841.3
(22) Date of filing: 04.03.2011
(51) Int. Cl.: C12N 1/22, C12N 1/18, C12P 7/10

(54) **Specific arabinose transporter of the plant arabidosis thaliana for the construction of pentose-fermenting yeasts**
Spezifische Arabinosetransporter der Arabidopsis thaliana-Pflanze zur Konstruktion von pentosefermentierenden Hefen
Transporteur d'arabinose spécifique de la plante arabidosis thaliana pour la construction de levures de fermentation à base de pentose

(43) Date of publication of application: 05.09.2012
(73) Proprietor: Butalco GmbH, 6352 Zürich (CH)
(72) Inventor: Boles, Eckhard, 64293 Darmstadt (DE); Subtil, Thorsten, 63110 Rodgau (DE)
(74) Representative: Engelhard, Markus

(56) References cited:
- WO-A1-2008/080505
- WO-A2-2007/136428
- BECKER JESSICA ET AL: "A MODIFIED SACCHAROMYCES CEREVISIAE STRAIN THAT CONSUMES L-ARABINOSE AND PRODUCES ETHANOL", APPLIED AND ENVIRONMENTAL MICROBIOLOGY, AMERICAN SOCIETY FOR MICROBIOLOGY, US, vol. 69, no. 7, 1 July 2003 (2003-07-01), pages 4144-4150, XP009080581, ISSN: 0099-2240, DOI: 10.1128/AEM.69.7.4144-4150.2003
- TANJA HAMACHER ET AL: "Characterization of the xylose-transporting properties of yeast hexose transporters and their influence on xylose utilization.", MICROBIOLOGY, vol. 148, no. 9, 1 September 2002 (2002-09-01), pages 2783-2788, XP55007165, ISSN: 1350-0872
- WIEDEMANN BEATE ET AL: "Codon-optimized bacterial genes improve L-Arabinose fermentation in recombinant Saccharomyces cerevisiae", APPLIED AND ENVIRONMENTAL MICROBIOLOGY, AMERICAN SOCIETY FOR MICROBIOLOGY, US, vol. 74, no. 7, 1 April 2008 (2008-04-01), pages 2043-2050, XP002494170, ISSN: 1098-5336, DOI: 10.1128/AEM.02395-07 [retrieved on 2008-02-08]

### SEQUENCE LISTING

<110> Johann Wolfgang Goethe Universität, Frankfurt am Main
<120> Specific arabinose transporter of the plant Arabidopsis thaliana for the construction of pentose-fermenting yeasts
<130> FB24419
<160> 2
<170> PatentIn version 3.3
<210> 1
   <211> 498
   <212> PRT
   <213> Arabidopsis thaliana
<400> 1
   Met Ala Val Gly Ser Met Asn Val Glu Glu Gly Thr Lys Ala Phe Pro
   1               5                   10                  15
   Ala Lys Leu Thr Gly Gln Val Phe Leu Cys Cys Val Ile Ala Ala Val
               20                  25                  30
   Gly Gly Leu Met Phe Gly Tyr Asp Ile Gly Ile Ser Gly Gly Val Thr
           35                  40                  45
   Ser Met Asp Thr Phe Leu Leu Asp Phe Phe Pro His Val Tyr Glu Lys
       50                  55                  60
   Lys His Arg Val His Glu Asn Asn Tyr Cys Lys Phe Asp Asp Gln Leu
   65                  70                  75                  80
   Leu Gln Leu Phe Thr Ser Ser Leu Tyr Leu Ala Gly Ile Phe Ala Ser
                   85                  90                  95
   Phe Ile Ser Ser Tyr Val Ser Arg Ala Phe Gly Arg Lys Pro Thr Ile
               100                 105                 110
   Met Leu Ala Ser Ile Phe Phe Leu Val Gly Ala Ile Leu Asn Leu Ser
           115                 120                 125
   Ala Gln Glu Leu Gly Met Leu Ile Gly Gly Arg Ile Leu Leu Gly Phe
       130                 135                 140
   Gly Ile Gly Phe Gly Asn Gln Thr Val Pro Leu Phe Ile Ser Glu Ile
   145                 150                 155                 160
   Ala Pro Ala Arg Tyr Arg Gly Gly Leu Asn Val Met Phe Gln Phe Leu
                   165                 170                 175
   Ile Thr Ile Gly Ile Leu Ala Ala Ser Tyr Val Asn Tyr Leu Thr Ser
               180                 185                 190
   Thr Leu Lys Asn Gly Trp Arg Tyr Ser Leu Gly Gly Ala Ala Val Pro
           195                 200                 205
   Ala Leu Ile Leu Leu Ile Gly Ser Phe Phe Ile His Glu Thr Pro Ala
       210                 215                 220
   Ser Leu Ile Glu Arg Gly Lys Asp Glu Lys Gly Lys Gln Val Leu Arg
   225                 230                 235                 240
   Lys Ile Arg Gly Ile Glu Asp Ile Glu Leu Glu Phe Asn Glu Ile Lys
                   245                 250                 255
   Tyr Ala Thr Glu Val Ala Thr Lys Val Lys Ser Pro Phe Lys Glu Leu
               260                 265                 270
   Phe Thr Lys Ser Glu Asn Arg Pro Pro Leu Val Cys Gly Thr Leu Leu
           275                 280                 285
   Gln Phe Phe Gln Gln Phe Thr Gly Ile Asn Val Val Met Phe Tyr Ala
       290                 295                 300
   Pro Val Leu Phe Gln Thr Met Gly Ser Gly Asp Asn Ala Ser Leu Ile
   305                 310                 315                 320
   Ser Thr Val Val Thr Asn Gly Val Asn Ala Ile Ala Thr Val Ile Ser
                   325                 330                 335
   Leu Leu Val Val Asp Phe Ala Gly Arg Arg Cys Leu Leu Met Glu Gly
               340                 345                 350
   Ala Leu Gln Met Thr Ala Thr Gln Met Thr Ile Gly Gly Ile Leu Leu
           355                 360                 365
   Ala His Leu Lys Leu Val Gly Pro Ile Thr Gly His Ala Val Pro Leu
       370                 375                 380
   Ile Val Leu Ile Leu Ile Cys Val Tyr Val Ser Gly Phe Ala Trp Ser
   385                 390                 395                 400
   Trp Gly Pro Leu Gly Trp Leu Val Pro Ser Glu Ile Tyr Pro Leu Glu
                   405                 410                 415
   Val Arg Asn Ala Gly Tyr Phe Cys Ala Val Ala Met Asn Met Val Cys
               420                 425                 430
   Thr Phe Ile Ile Gly Gln Phe Phe Leu Ser Ala Leu Cys Arg Phe Arg
   435 440 445
   Ser Leu Leu Phe Phe Phe Phe Gly Ile Met Asn Ile Ile Met Gly Leu
   450 455 460
   Phe Val Val Phe Phe Leu Pro Glu Thr Lys Gly Val Pro Ile Glu Glu
   465 470 475 480
   Met Ala Glu Lys Arg Trp Lys Thr His Pro Arg Trp Lys Lys Tyr Phe
   485 490 495
   Lys Asp
<210> 2
   <211> 1497
   <212> DNA
   <213> Arabidopsis thaliana
<400> 2
   atggctgttg gttcgatgaa tgtcgaagaa ggcactaaag cttttcctgc gaagctgaca     60
   ggtcaagtgt ttctttgctg tgtcattgct gctgttggtg gtctcatgtt tggttacgac    120
   atcggaatct caggaggtgt gacgagtatg gacactttct tgttagattt tttcccacac    180
   gtgtacgaga agaaacacag agtacacgaa aacaactact gcaaattcga tgaccagctc    240
   ttgcagttgt tcacttcctc actctacttg gctggaatct tcgctagctt tatttcttcc    300
   tatgtttcta gggcttttgg aagaaaaccc acaatcatgc tcgcctccat cttcttcctc    360
   gtcggtgcta tcctcaactt atctgcccaa gaactcggca tgttgatcgg tggtcgtatt    420
   cttctcggct ttggtatcgg tttcggtaac cagaccgttc cattgttcat ctcagagatt    480
   gctccggcga gatacagagg aggactaaac gtcatgttcc agtttctcat caccattgga    540
   atcttagcag caagttatgt gaactactta acttccacgt tgaaaaacgg ctggagatat    600
   tctctcggtg gtgctgctgt ccccgcttta atcctcttga taggatcctt ctttatccac    660
   gagactccgg ctagtctcat cgagcgcggt aaagacgaaa aggggaagca agtcctgagg    720
   aagatcagag gcattgaaga tattgagctc gagttcaatg agatcaaata cgcaacagag    780
   gtagcgacaa aggtgaaaag cccattcaaa gaactcttca ccaagagtga gaacagacca    840
   ccattggtgt gtggaacgtt acttcagttc tttcaacagt tcaccggtat caacgtggtt    900
   atgttctacg ctccagtttt gttccagacg atgggtagtg gtgacaacgc ttctctgatc    960
   tctactgttg tcaccaatgg tgtgaacgca atcgctacag ttatctctct tttagtggtt   1020
   gatttcgccg gtagaaggtg tcttctgatg gaaggagctc tccagatgac cgctacacag   1080
   atgacaattg gaggcattct cttagctcac ttgaagctag ttggtcctat tactggccat   1140
   gccgtgccgc tgattgtatt gatcctcatc tgcgtatacg tgtctggatt tgcatggtct   1200
   tggggtccat tgggatggct tgttccgtct gagatctacc ctcttgaagt gagaaatgct   1260
   ggttacttct gtgcggtggc gatgaacatg gtctgcactt tcatcattgg acagttcttc   1320
   ttgtctgctc tctgcagatt cagatcatta ttgttcttct tcttcggaat aatgaacatt   1380
   atcatgggac tatttgtcgt gtttttcttt cccgaaacca aaggtgttcc tattgaggaa   1440
   atggccgaga agcgttggaa aacgcacccg cgttggaaga aatatttcaa agactag      1497

## Description

The present invention relates to the use of nucleic acid molecules coding for a plant pentose transporter, preferably from *Arabidopsis thaliana,* for the transformation of a yeast cell, wherein the transformation enables the yeast cell to specifically take up L-arabinose, and, thus for the conversion/metabolization, particularly fermentation, of biomaterial containing pentose(s), in particular arabinose, with recombinant microorganisms, and particularly for the production of bio-based chemicals and biofuels, in particular bioethanol, by means of arabinose-fermenting yeasts. The present invention further relates to yeast cells, which are transformed with a nucleic acid expression construct, which codes for a plant pentose transporter, wherein the expression of the nucleic acid expression construct imparts to the cells the capability to take up L-arabinose. Said cells are preferably utilized for the conversion/metabolization, particularly fermentation, of biomaterial containing arabinose, and particularly for the production of bio-based chemicals and biofuels, in particular bioethanol. The present invention also relates to methods for the production of bio-based chemicals and biofuels, in particular bioethanol.

### BACKGROUND OF THE INVENTION

The beer, wine and baking yeast *Saccharomyces cerevisiae* has already been used for centuries for the production of bread, wine and beer owing to its characteristic of fermenting sugar to ethanol and carbon dioxide. In biotechnology, *S.cerevisiae* is used particularly in ethanol production for industrial purposes, in addition to the production of heterologous proteins. Ethanol is used in numerous branches of industry as an initial substrate for syntheses. Ethanol is gaining increasing importance as an alternative fuel, due to the increasingly scarce presence of oil, the rising oil prices and continuously increasing need for petrol worldwide.

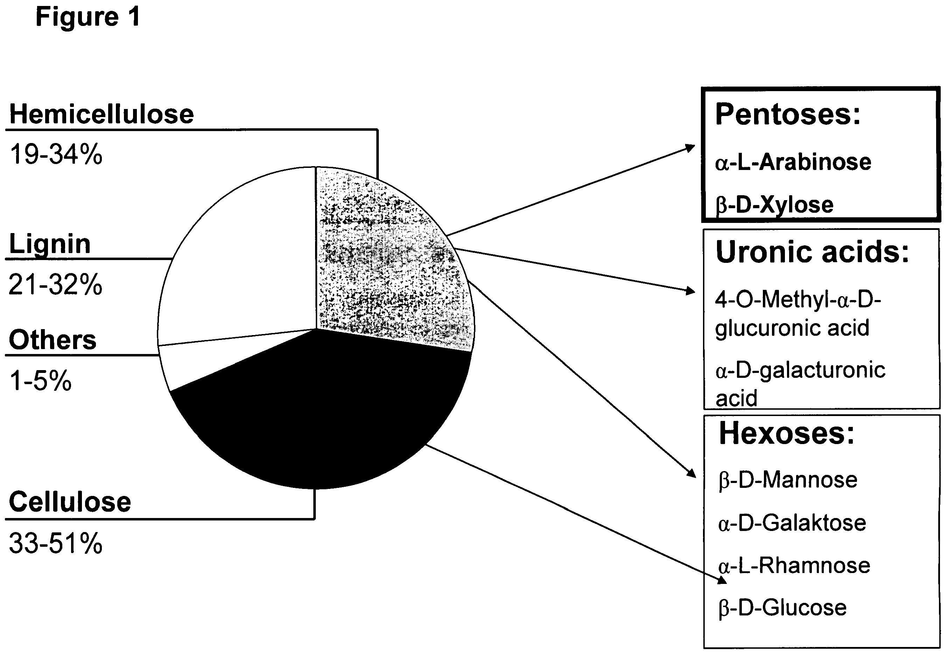

In order to make possible a favourably-priced and efficient bioethanol production, the use of biomass containing lignocellulose, such as for example straw, waste from the timber industry and agriculture and the organic component of everyday household waste, presents itself as an initial substrate. Firstly, said biomass is very convenient and secondly is present in large quantities. The three major components of lignocellulose are lignin, cellulose and hemicellulose. Hemicellulose, which is the second most frequently occurring polymer after cellulose, is a highly branched heteropolymer. It consists of pentoses (L-arabinose, D-xylose), uronic acids (4-O-methyl-D-glucuronic acid, D-galacturonic acid) and hexoses (D-mannose, D-galactose, L-rhamnose, D-glucose) (see Figure 1). Although, hemicellulose can be hydrolized more easily than cellulose, it contains the pentoses L-arabinose and D-xylose, which can normally not be converted by the yeast *S.cerevisae.*

In order to be able to use pentoses for fermentations, these must firstly enter the cell through the plasma membrane. Although *S.cerevisiae* is not able to metabolize D-xylose, it can uptake D-xylose into the cell. However, *S.cerevisiae* does not have a specific transporter. The transport takes place by means of the numerous hexose transporters. The affinity of the transporters to D-xylose is, however, distinctly lower than to D-glucose (Kotter and Ciriacy, 1993). In yeasts which are able to metabolize D-xylose, such as for example *P.stipitis, C.shehatae* or *P.tannophilus* (Du Preez et al., 1986), there are both unspecific low-affinity transporters, which transport D-glucose, and also specific high-affinity proton symporters only for D-xylose (Hahn-Hagerdal *et al.,* 2001).

In earlier experiments, some yeasts were found, such as for example *Candida tropicalis, Pachysolen tannophilus, Pichia stipitis, Candida shehatae,* which by nature ferment L-arabinose or can at least assimilate it. However, these yeasts lack entirely the capability of fermenting L-arabinose to ethanol, or they only have a very low ethanol yield (Dien et al., 1996). Moreover, very little is yet known about the uptake of L-arabinose. In the yeast *C.shehatae* one assumes a proton symport (Lucas and Uden, 1986). In *S.cerevisiae,* it is known from the galactose permease Gal2 that it also transports L-arabinose, which is very similar in structure to D-galactose. (Kou *et al.,* 1970).

Alcoholic fermentation of pentoses in biotechnologically modified yeast strains of *S.cerevisiae,* wherein inter alia various genes of the yeast strain *Pichia stipitis* were used for the genetic modification of *S.cerevisiae,* was described in recent years particularly in connection with the fermentation of xylose. The engineering concentrated here particularly on the introduction of the genes for the initial xylose assimilation from *Pichia stipitis,* a xylose-fermenting yeast, into *S.cerevisiae*, i.e. into a yeast which is traditionally used in the ethanol production from hexose (Jin et al. 2004).

Jeppson et al. (2006) describe xylose fermentation by *S.cerevisiae* by means of the introduction of a xylose metabolic pathway which is either similar to that in the yeasts *Pichia stipitis* and *Candida shehatae*, which naturally use xylose, or is similar to the bacterial metabolic pathway.

Katahira et al. (2006) describe sulphuric acid hydrolysates of lignocellulose biomass such as wood chips, as an important material for the production of fuel bioethanol. In this study, a recombinant yeast strain was constructed, which is able to ferment xylose and cellooligosaccharides. For this, various genes were integrated into this yeast strain and namely for the inter-cellular expression of xylose reductase and xylitol dehydrogenase from *Pichia stipitis* and xylulokinase from *S.cerevisiae* and for the presentation of beta-glucosidase from *Aspergillus acleatus* on the cell surface. In the fermentation of sulphuric acid hydrolysates of wood chips, xylose and cellooligosaccharides were fully fermented by the recombinant strain after 36 hours.

Pitkanen et al. (2005) describe the obtaining and characterizing of xylose chemostat isolates of a *S.cervisiae* strain, which over-expresses genes of *Pichia stipitis* coding for xylose reductase and xylitol dehydrogenase and the gene which codes endogenous xylulokinase. The isolates were obtained from aerobic chemostat cultures on xylose as the single or major carbon source. Under aerobic conditions on minimal medium with 30 g/l xylose, the growth rate of the chemostat isolates was 3 times higher than that of the original strain (0.15 h⁻¹ compared with 0.05 h⁻¹). The xylose uptake rate was increased almost two-fold. The activities of the key enzymes of the pentose phosphate metabolic pathway (transketolase, transaldolase) were increased two-fold, whilst the concentrations of their substrates (pentose-5-phosphates, sedoheptulose-7-phosphate) were lowered accordingly.

Becker and Boles (2003) describe the engineering and the selection of a laboratory strain of *S.cerevisiae* which is able to use L-arabinose for growth and for fermenting it to ethanol. This was possible due to the over-expression of a bacterial L-arabinose metabolic pathway, consisting of *Bacillus subtilis* AraA and *Escherichia coli* AraB and AraD and simultaneous over-expression of yeast galactose permease transporting L-arabinose in the yeast strain.

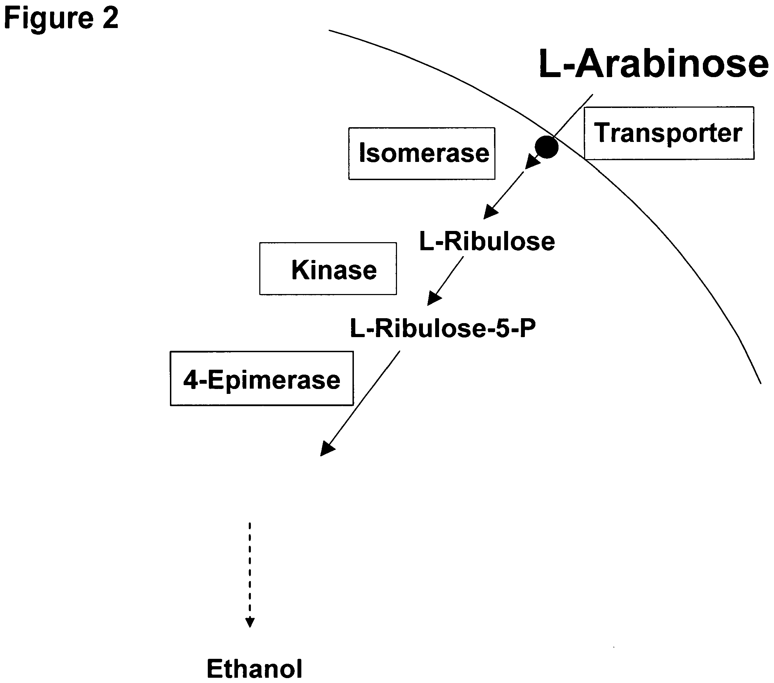

Molecular analysis of the selected strain showed that the predetermining precondition for a use of L-arabinose is a lower activity of L-ribulokinase. However, inter alia, a very slow growth is reported from this yeast strain (see Figure 2).
Wiedemann and Boles (2008) show that expressing of the codon-optimized genes of L-arabinose isomerase from *Bacillus licheniformis* and L-ribulokinase and L-ribulose-5-P 4-epimerase from *Escherichia coli* strongly improved L-arabinose conversion rates.
WO 2008/080505 discloses an arabinose transporter from *Pichia stipitis,* which enables yeast cells to take up L-arabinose.
There still exists a need in the art for specific pentose transporters, in particular specific L-arabinose transporters, which allow to specifically take up L-arabinose into cells, such as yeast cells, and therefore to promote the utilization and fermentation of pentoses, in particular L-arabinose.
It is thus an object of the present invention to provide improved and more specific pentose transporters, such as L-arabinose transporters.
The object is solved according to the invention as it is claimed in the claims.

### Plant pentose transporter constructs and their use

Disclosed is a nucleic acid molecule comprising a nucleic acid sequence, which codes for a plant pentose transporter, for
- the transformation of a yeast cell, preferably for the recombinant expression and production of the plant pentose transporter,
- the specific uptake of L-arabinose by the yeast cell, and/or
- the conversion of L-arabinose by the yeast cell resulting in the formation of secondary products.
In particular for the following uses:
- the transformation of a yeast cell, preferably for the recombinant expression and production of the plant pentose transporter in said yeast cell,
- the conversion and metabolization, particularly recombinant fermentation, of biomaterial containing L-arabinose,
- the production of bio-based chemicals or biofuels,
- the production of bioalcohols, preferably bioethanol and/or biobutanol.
"Secondary products" refer to those compounds, which the cell further produces from L-arabinose after the cell has taken up the L-arabinose, such as, for example, bio-based chemicals and bioalcohols.
"Bio-based chemicals" or "biofuels" refer to chemical compounds and substances, which are obtained from biological materials and raw materials (biomass), particularly by using microorganisms.
The bio-based chemicals or biofuels can be compounds, which are selected from, but not limited to: lactic acid, acetic acid, succinic acid, malic acid, 1-butanol, isobutanol, 2-butanol, other alcohols, amino acids, 1,3-propanediol, ethylene, glycerine, a β-lactam antibiotic or a cephalosporin, alkanes, terpenes, isoprenoids or the precursor molecule amorphadiene of the antimalarial drug artemisinin.
The terms "conversion" and "metabolization" are used synonymously and refer to the metabolism of a substance or the conversion of a substance in the course of the metabolism, here: the conversion of L-arabinose by a cell, which was transformed with a nucleic acid according to the invention. A preferred conversion/metabolization is fermentation, in particular recombinant fermentation.
The nucleic acid molecules used according to the invention are recombinant nucleic acid molecules. Furthermore, nucleic acid molecules used according to the disclosure comprise dsDNA, ssDNA, PNA, CNA, RNA or mRNA or combinations thereof.
The plant pentose transporter according to the invention originates from the plant *Arabidopsis*, preferably *Arabidopsis thaliana.*
The plant pentose transporter is preferably the transporter Stp2 from *Arabidopsis thaliana.* Stp2 is a protein of 498 amino acids (see SEQ ID NO. 1).

In this invention, a specific L-arabinose transporter gene from *A. thaliana* was identified by using a test system (see examples). Truernit et al., 1999 identified the transporter Stp2 from *Arabidopsis thaliana* as a proton symporter with a high affinity for galactose. The inventors designed and used the plasmid pTHStp2, which has the ORF from *AtSTP2* localized on it. Yeast cells of the strain MKY06 were transformed with the plasmid pTHStp2, which is responsible for a specific growth on L-arabinose but not on D-glucose. The possibility that the obtained growth of the transformed yeast cells was brought about by a genomic mutation in MKY06 was ruled out. After a selection on the loss of the plasmid pTHStp2 no further growth was established with renewed smearing on L-arabinose medium.

Thus, the plant pentose transporter used according to the disclosure allows the specific *in vitro* and/or *in vivo* transport and uptake of L-arabinose into the transformed yeast cell, however, it does not transport D-glucose. This allows a respective transformed yeast cell or strain to convert and metabolize, particularly recombinantly ferment, biomaterial which contains pentose(s) (preferably L-arabinose), but also biomaterial which contains hexoses and pentoses, preferably D-glucose, D-xylose and L-arabinose. Wherein the respective transformed yeast cell or strain is imparted the capability to take up L-arabinose in the presence of hexoses, particularly D-glucose.
Due to the specificity of the plant pentose transporter used according to the invention, after expression in existing ethanol-producing systems, such as respective recombinant yeast cells/strains, the uptake rate for L-arabinose can be improved, because firstly with high L-arabinose concentrations the competitive situation with respect to glucose is improved, and secondly with low L-arabinose concentrations the transport of L-arabinose becomes more efficient owing to the high affinity.

The plant pentose transporter according to the disclosure comprises an amino acid sequence, which is at least 70% identical, preferably at least 80% identical, more preferably at least 90% identical, even more preferably at least 95% identical and yet more preferably 99% identical to the amino acid sequence of SEQ ID NO: 1 and has an *in vitro* and/or *in vivo* pentose transport function (in particular an *in vitro* and/or *in vivo* L-arabinose transport function) or is identical to the amino acid sequence of SEQ ID NO: 1.
As used herein, the term "percent (%) identical" refers to sequence identity between two amino acid sequences. Identity can be determined by comparing a position in both sequences, which may be aligned for the purpose of comparison. When an equivalent position in the compared sequences is occupied by the same amino acid, the molecules are considered to be identical at that position.
As used herein, the term "functional equivalent" refers to amino acid sequences that are not 100% identical to the amino acid sequence of SEQ ID NO. 1 and comprise amino acid additions and/or insertions and/or deletions and/or substitutions and/or exchanges, which do not alter or change the activity or function of the protein as compared to the protein having the amino acid sequence of SEQ ID NO: 1, i.e. an "functional equivalent", for example, encompasses an amino acid sequence with conservative amino acid substitutions or smaller deletions and/or insertions as long as these modifications do not substantially affect the *in vitro* and/or *in vivo* L-arabinose transport function.
Generally, a person skilled in the art is aware of the fact that some amino acid exchanges in the amino acid sequence of a protein do not have an influence on the (secondary or tertiary) structure, function and/or activity of that protein. Amino acid sequences with such "neutral" amino acid exchanges as compared to the amino acid sequences are disclosed herein. When the nucleic acid sequence coding for the plant pentose transporter is expressed in a yeast cell, the yeast cell is imparted the capability to take up L-arabinose, which then may be metabolized further. Through this, the cell is able to grow on L-arabinose as a carbon source.
The nucleic acid sequence coding for the plant pentose transporter preferably comprises a nucleic acid sequence, which is at least 70% identical, preferably at least 80% identical, more preferably at least 90% identical, even more preferably at least 95% identical and yet more preferably 99% identical or identical to the nucleic acid sequence of SEQ ID NO: 2.

The nucleic acid molecules according to the disclosure preferably comprise nucleic acid sequences, which are identical with the naturally occurring nucleic acid sequence or are codon-optimized for the use in a host cell.

The nucleic acid molecule used according to the present disclosure is preferably a nucleic acid expression construct.

Nucleic acid expression constructs according to the invention are expression cassettes comprising a nucleic acid molecule according to the invention, or expression vectors comprising a nucleic acid molecule according to the invention or an expression cassette, for example.

A nucleic acid expression construct preferably comprises regulatory sequences, such as promoter and terminator sequences, which are operatively linked with the nucleic acid sequence coding for the plant pentose transporter.

Preferred promoter sequences are HXT7, truncated HXT7, PFK1, FBA1, PGK1, ADH1 and TDH3. Preferred terminator sequences are CYC1, FBA1, PGK1, PFK1, ADH1 and TDH3.

The nucleic acid expression construct may further comprise 5' and/or 3' recognition sequences and/or selection markers.

A preferred selection marker is a LEU2 marker gene, a URA3 marker gene, a TRP1 marker gene, a HIS3 marker gene and a dominant antibiotic-resistance marker gene. A preferred dominant antibiotic-resistance marker gene is a gene, which imparts resistances to geneticin, hygromycin and nourseothricin.

The yeast cell is preferably a member of a genus selected from the group of *Saccharomyces, Kluyveromyces, Candida, Pichia, Schizosaccharomyces, Hansenula, Kloeckera, Schwanniomyces, Arxula* and *Yarrowia.*

The yeast cell is more preferably a member of a species selected from the group of *S. cerevisiae, S. bulderi, S. barnetti, S. exiguus, S. uvarum, S. diastaticus, K. lactis, K. marxianus, K. fragilis, H.polymorpha, P.pastoris* and *Y.lipolytica,* such as *S. cerevisiae, K. lactis, H.polymorpha, P.pastoris* or *Y.lipolytica.*

In a preferred embodiment, the yeast cell further contains nucleic acid molecules which code for proteins of an arabinose metabolic pathway, in particular for L-ribulokinase, L-ribulose-5-P 4-epimerase, L-arabinose-isomerase.

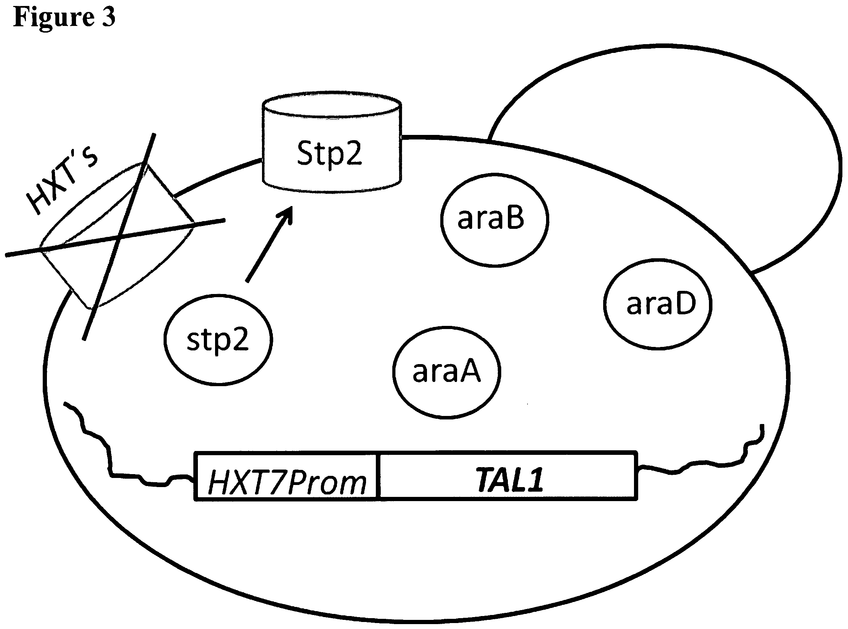

Preferred are proteins of the bacterial arabinose metabolic pathway, in particular *E.coli araB* L-ribulokinase, *E.coli araD* L-ribulose-5-P 4-epimerase and *B.licheniformis araA* L-arabinose-isomerase. See also Figures 2 and 3.

Depending on the intended use of the yeast cell, said yeast cell can contain, express or overexpress further nucleic acid sequences coding for further proteins, such as transaldolase TAL1 and/or TAL2, transketolase TKL1 and/or TKL2, D-ribulose-5-phosphate 3-epimerase RPE1, ribose-5-phosphate ketol-isomerase RKI1 or the corresponding sequences from other organisms encoding the same enzyme activities.

According to the invention, a yeast cell according to this invention is modified by the introduction and expression of the genes araA (L-arabinose-isomerase), araB (L-ribulokinase) and araD (L-ribulose-5-P-4-epimerase) and in addition over-expresses a TAL1 (transaldolase) gene, as described for example by the inventors in EP 1 499 708 B1, and in addition to this contains at least one nucleic acid molecule according to the invention.

### Yeast cells specifically taking up L-arabinose

The object is solved according to the invention by providing a yeast cell which is transformed with a nucleic acid expression construct comprising:
(a) a nucleic acid sequence coding for a plant pentose transporter, wherein the plant pentose transporter comprises an amino acid sequence, which is at least 70%, preferably at least 80%, more preferably at least 90% identical to the amino acid sequence of SEQ ID NO: 1 and has an *in vitro* and/or *in vivo* pentose transport function,
(b) regulatory elements operatively linked with the nucleic acid sequence, allowing for the expression of the plant pentose transporter in the yeast cell,
wherein the expression of the nucleic acid expression construct imparts to the yeast cell the capability to take up L-arabinose.

Preferably, the expression of the nucleic acid expression construct imparts to the yeast cell the capability to take up L-arabinose in the presence of hexoses, particularly D-glucose. According to the invention, the yeast cell of the present invention over-expresses a TAL1 (transaldolase) gene and expresses the genes araA (L-arabinose-isomerase), araB (L-ribulokinase) and araD (L-ribulose-5-P-4-epimerase).

As disclosed above, the plant pentose transporter used according to the disclosure allows the specific *in vitro* and/or *in vivo* transport and uptake of L-arabinose into a transformed yeast cell, however, it does not transport D-glucose. This allows the respective transformed yeast cell or strain to convert and metabolize, particularly recombinantly ferment, biomaterial which contains pentose(s) (namely L-arabinose), but also biomaterial which contains hexoses and pentoses, preferably D-glucose, D-xylose and L-arabinose. Wherein the respective transformed yeast cell or strain is imparted the capability to take up L-arabinose in the presence of hexoses, particularly D-glucose.
Due to the specificity of the plant pentose transporter used according to the invention, after expression in existing ethanol-producing systems, such as respective recombinant yeast cells/strains, the uptake rate for L-arabinose can be improved, because firstly with high L-arabinose concentrations the competitive situation with respect to glucose is improved, and secondly with low L-arabinose concentrations the transport of L-arabinose becomes more efficient owing to the high affinity.
The plant pentose transporter according to the invention originates from *Arabidopsis*, preferably *Arabidopsis thaliana.* The plant pentose transporter according to the invention comprises an amino acid sequence, which is at least 70% identical, preferably at least 80% identical, more preferably at least 90% identical, even more preferably at least 95% identical and yet more preferably 99% identical and has an *in vitro* and/or *in vivo* pentose transport function (in particular an *in vitro* and/or *in vivo* L-arabinose transport function), as disclosed and defined herein, or is identical to the amino acid sequence of SEQ ID NO: 1, as disclosed and defined herein.
The regulatory elements, such as promoter and terminator sequences, are operatively linked with the nucleic acid sequence coding for the plant pentose transporter and allow for the expression of the plant pentose transporter in the yeast cell.
Preferred promoter sequences are HXT7, truncated HXT7, PFK1, FBA1, PGK1, ADH1 and TDH3. Preferred terminator sequences are CYC1, FBA1, PGK1, PFK1, ADH1 and TDH3.
The nucleic acid expression construct may further comprise 5' and/or 3' recognition sequences and/or selection markers. A preferred selection marker is a LEU2 marker gene, a URA3 marker gene, a TRP1 marker gene, a HIS3 marker gene and a dominant antibiotic-resistance marker gene. A preferred dominant antibiotic-resistance marker gene is a gene, which imparts resistances to geneticin, hygromycin and nourseothricin.
In a preferred embodiment, the nucleic acid expression construct with which a yeast cell according to the invention is transformed is a nucleic acid molecule according to the invention, as defined herein and above.
The yeast cell is preferably a member of a genus selected from the group of *Saccharomyces, Kluyveromyces, Candida, Pichia, Schizosaccharomyces, Hansenula, Kloeckera, Schwanniomyces, Arxula* and *Yarrowia.*
The yeast cell is more preferably a member of a species selected from the group of *S. cerevisiae, S. bulderi, S. barnetti, S. exiguus, S. uvarum, S. diastaticus, K. lactis, K. marxianus, K. fragilis, H.polymorpha, P.pastoris* and *Y.lipolytica,* such as *S. cerevisiae, K. lactis, H.polymorpha, P.pastoris* or *Y.lipolytica.*

According to the disclosure, the yeast cell further contains nucleic acid molecules which code for proteins of an arabinose metabolic pathway, in particular for L-ribulokinase, L-ribulose-5-P 4-epimerase, L-arabinose-isomerase.
Preferred are proteins of the bacterial arabinose metabolic pathway, in particular *E.coli araB* L-ribulokinase, *E.coli araD* L-ribulose-5-P 4-epimerase and *B.licheniformis araA* L-arabinose-isomerase. See also Figures 2 and 3.
Depending on the intended use of the yeast cell, said yeast cell can contain, express or overexpress further nucleic acid sequences coding for further proteins, such as transaldolase TAL2, transketolase TKL1 and/or TKL2, D-ribulose-5-phosphate 3-epimerase RPE1, Ribose-5-phosphate ketol-isomerase RKI1 or the corresponding sequences from other organisms encoding the same enzyme activities.
According to the invention, a yeast cell according to this invention is modified by the introduction and expression of the genes araA (L-arabinose-isomerase), araB (L-ribulokinase) and araD (L-ribulose-5-P-4-epimerase) and in addition over-expresses a TAL1 (transaldolase) gene, as described for example by the inventors in EP 1 499 708 B1, and in addition to this contains at least one nucleic acid molecule according to the invention.

A yeast cell according to the disclosure is more preferably the strain Ethanol Red™ or Lallemand1 or other yeast strains commonly used in the bioethanol industry.

The yeast cell according to the disclosure is preferably a cell maintained in a cell culture or a cultured cell.

The yeast cells according to the disclosure are transiently or stably transformed with the nucleic acid expression construct or the nucleic acid molecule, as defined herein.
In one embodiment, a yeast cell according to the invention furthermore expresses one or more enzymes, which impart to the cell the capability to produce one or more further metabolization products.
A "further metabolization product" is preferably selected from, but not limited to, the group of bio-based chemicals or biofuels, such as lactic acid, acetic acid, succinic acid, malic acid, 1-butanol, isobutanol, 2-butanol, other alcohols, amino acids, 1,3-propanediol, ethylene, glycerol, a β-lactam antibiotic or a cephalosporin, alkanes, terpenes, isoprenoids or the precursor molecule amorphadiene of the antimalarial drug artemisinin.
The object is solved according to the invention by using the yeast cells according to the invention for
- the conversion and metabolization, particularly recombinant fermentation, of biomaterial containing L-arabinose,
- the production of bio-based chemicals or biofuels,
- the production of bioalcohols, preferably bioethanol and/or biobutanol.

### Methods for the production of biomolecules

The object is furthermore solved according to the invention by providing a method for the production of *bioalcohol(s).*
The method according to the invention comprises the following steps:
(a) converting a medium containing an L-arabinose source with a cell according to the invention, which converts L-arabinose to a bioalcohol,
(b) optionally obtaining the bioalcohol,

The bioalcohol is preferably bioethanol and/or biobutanol. The bioalcohol is obtained by isolation, for example.

The medium may also contain a further carbon source, particularly hexose, more particularly glucose.

The object is furthermore solved according to the invention by providing a method for the production of *metabolization product(s)* / *bio-based chemicals or biofuels.*

The method according to the invention comprises the following steps:
(a) converting a medium containing an L-arabinose source with a cell according to claim 13, which converts L-arabinose to produce the metabolization product,
(b) optionally obtaining the metabolization product,

The metabolization product is preferably selected from the group of bio-based chemicals or biofuels, such as lactic acid, acetic acid, succinic acid, malic acid, 1-butanol, isobutanol, 2-butanol, other alcohols, amino acids, 1,3-propanediol, ethylene, glycerol, a β-lactam antibiotic or a cephalosporin, alkanes, terpenes, isoprenoids or the precursor molecule amorphadiene of the antimalarial drug artemisinin.

The metabolization product is obtained by isolation, for example.

The medium may also contain a further carbon source, particularly hexose, more particularly glucose.

The inventors have identified by a gene screening a novel specific L-arabinose transporter, the nucleotide and protein sequence of which is presented herein (see SEQ ID NOs: 1 and 2). For this, reference is also to be made to the examples and figures.

Due to the specificity of this novel transporter, after expression in existing ethanol-producing systems the uptake rate for L-arabinose can be improved, because firstly with high L-arabinose concentrations the competitive situation with respect to glucose is improved, and secondly with low L-arabinose concentrations the transport of L-arabinose becomes more efficient owing to a high affinity.

### Uptake of L-arabinose

So that the pentose L-arabinose can be metabolized by *S.cerevisiae*, it must firstly be taken up by the cell. Only little is known with regard to this uptake. Hitherto, no genes are known in eukaryontes, which code for specific L-arabinose transporters. All hexose transporters tested for the pentose D-xylose have a much higher affinity to hexoses than to D-xylose. For L-arabinose, a similar situation is assumed. Of all strains constructed hitherto, which can utilize pentoses (D-xylose or L-arabinose), a relatively slow growth is reported. Above all, the slow and poor uptake of the pentoses is named as a reason for this (Becker and Boles, 2003; Richard *et al.,* 2002). In fermentations in a sugar mixture, consisting of D-glucose and D-xylose or D-glucose and L-arabinose, the sugars are not converted simultaneously. Due to the high affinity of the transporters for D-glucose, D-glucose is metabolized at first. A so-called Diauxic shift occurs. Only after the D-glucose is exhausted is the pentose converted in a second, distinctly slower growth phase (Kuyper *et al.,* 2005a; Kuyper *et al.*, 2005b). The absence of specific transporters for pentoses is given as an explanation.

### Novel specific L-arabinose transporter from A. thaliana

For industrial applications, it would be ideal if the microorganism which was used could convert all the sugars present in the medium as far as possible simultaneously (Zaldivar *et al.,* 2001). In order to achieve this, specific transporters for each sugar type would be of great benefit. None were known hitherto particularly for L-arabinose.

In this invention, it has been successful with a test system (see examples) to find a specific L-arabinose transporter gene from *A. thaliana.* Truernit et al., 1999 identified the transporter Stp2 from *Arabidopsis thaliana* as a proton symporter with a high affinity for galactose. Truernit et al. furthermore characterized Stp2 in the yeast *Schizosaccharomyces pombe,* where the following sugars were transported: D-glucose, D-galactose, D-xylose, D-mannose, 3-O-methylglucose and D-fructose. The inventors used the plasmid pTHStp2, containing the ORF from *AtSTP2,* and found it to be responsible for a specific growth of respective transformed *S*. *cerevisiae* (strain MKY06) on L-arabinose but not on D-glucose, and in a comparable growth-based screen with overexpression of a xylose isomerase also not on xylose. The possibility that the obtained growth was brought about by a genomic mutation in MKY06 was ruled out. After a selection on the loss of the plasmid pTHStp2 no further growth was established with renewed smearing on L-arabinose medium. The results demonstrate that AtStp2 is able to confer to the yeast cells the property to take up arabinose with a higher specificity and with a higher affinity than normal yeast cells do. This property is important for the fermentation in particular of mixtures of hexose and pentose sugars, in particular D-glucose, D-xylose and L-arabinose, and in particular of plant biomass hydrolysates where normally D-glucose is present in much higher concentrations than L-arabinose. The specific properties of AtStp2 in the yeast cells will allow the fermentation of L-arabinose in the presence of D-glucose, and even at low L-arabinose concentrations.

Recently, Verho et al., 2011 describe the cloning of two genes LAT1 and LAT2 of the L-arabinose fermenting yeast *Ambrosiozyma monospora.* In the present inventors' test system, these genes, however, did not proof to be coding for arabinose transporters.

Furthermore, a multitude of experimental obstacles and difficulties were to be overcome in the locating and provision of the transporter according to the invention, which can also be seen in greater detail from the examples and figures.
- In the initial strain EBY.VW4000, a total of 21 monosaccharide transporter genes had to be deleted.
- Furthermore, in this strain *TALI* had to be over-expressed genomically.
- The establishing of the optimum growth conditions for carrying out the screen proved to be very difficult and time-consuming.
- The transporter according to the invention is the second described specific arabinose transporter of eukaryots. It has a higher specificity for arabinose than the transporter AraT of *P.stipitis* (as disclosed in WO 2008/080505) because it transports no glucose in the strain MKY06-3P in contrast to AraT which transports small amounts of glucose when overexpressed.
- The concern is with a heterologously expressed transporter which is at the same time functionally incorporated in the plasma membrane of *S.cerevisiae*, which is not implicitly to be expected.

Some reports exist with regard to the difficulties concerning heterologously expressed transporters, see on this subject Chapter 2 in the book "Transmembrane Transporters" (Boles, 2002) and the article by Wieczorke et al., 2003.

Further biomass with significant amounts of arabinose (source of the data: U.S. Department of Energy http://www.eere.energy.gov/biomass/progs/search1.cgi):

| Type of biomass | L-arabinose [%] |
|---|---|
| Switchgrass | 3.66 |
| Large bothriochloa | 3.55 |
| Tall fescue | 3.19 |
| Robinia | 3 |
| Corn stover | 2.69 |
| Wheat straw | 2.35 |
| Sugar can bagasse | 2.06 |
| Chinese lespedeza | 1.75 |
| Sorghum bicolor | 1.65 |

The arabinose transporter according to the invention is also of great importance for its utilization.

Possibilities for use of a functional and at the same time specific arabinose transporter in the yeast *S.cerevisiae* are firstly the production of bioethanol and the production of high-grade precursor products for further chemical syntheses.

The following list originates from the study "Top Value Added Chemicals From Biomass" (see www1.eere.energy.gov/biomass/pdfs/35523.pdf). Here, 30 chemicals were categorized as being particularly valuable, which can be produced from biomass.

| Number of C atoms | Top 30 Candidates |
|---|---|
| 1 | hydrogen, carbon monoxide |
| 2 | |
| 3 | glycerol, 3-hydroxypropionic acid, lactic acid, malonic acid, propionic acid, serine |
| 4 | acetoin, asparaginic acid, fumaric acid, 3-hydroxybutyrolactone, malic acid, succinic acid, threonin |
| 5 | arabitol, furfural, glutamic acid, itaconic acid, levulinic acid, proline, xylitol, xylonic acid |
| 6 | aconitic acid, citrate, 2,5-furandicarboxylic acid, glucaric acid, lysine, levoglucosan, sorbitol |

As soon as these chemicals are produced from lignocelluloses by bioconversion (e.g. fermentations with yeasts), it is important to have a specific transporter for the hemicellulose arabinose.

The present invention is further clarified in the following figures, sequences and examples, without however being restricted thereto. The cited references are fully included herewith by reference. In the sequences and figures there are shown:
SEQ ID NO: 1 the protein sequence of the Stp2,
SEQ ID NO: 2 the nucleotide sequence of the open reading frame (ORF) of Stp2.

**Figure 1****.** *Composition of the biomass*
   The second most frequently occurring hemicellulose is a highly branched polymer consisting of pentoses, uronic acids and hexoses. The hemicellulose consists in a large proportion of the pentoses xylose and arabinose.
**Figure 2****.** *Scheme for the use of L-arabinose in recombinant S.cerevisiae by integration of a bacterial L-arabinose metabolic pathway.*
**Figure 3****.** *Construction of the yeast strain MKY06-3P* + *pTHStp2 according to the invention.*
   The initial strain for the construction of MKY06-3P was the yeast strain EBY.VW4000, in which all hexose transporter genes (HXTs) were deleted. In this strain, the endogenous transaldolase *TAL1* was over-expressed by the exchange of the native promoter for the shortened *HXT7* promoter (*HXT7*-*Prom*). This led to the strain MKY06. Into this strain, the plasmids p423H7-synthIso, p424H7-synthKin and p425H7-synthEpi were transformed for the arabinose metabolism (=MKY06-3P). In addition, the plasmid pTHStp2 (*At*Stp2), which codes for the arabinose transporter according to the invention from *Arabidopsis thaliana* was also transformed into this strain and, thus, the strain MKY06-3P + pTHStp2 was obtained. The transporter is expressed and is functionally incorporated into the plasma membrane.

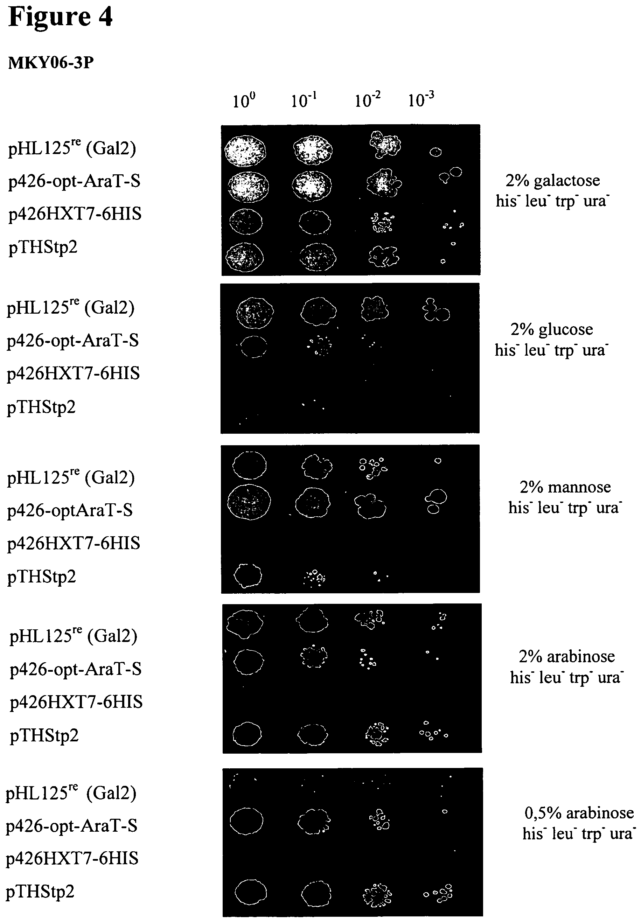

**Figure 4****.** *Dropping of the MKY06-3P with the plasmids for the L-arabinose metabolism and the found L-arabinose transporters on various carbon sources.*
   As a negative control (-), instead of the pTHStp2 plasmid, the p426HXT7-6HIS was transformed in as a negative control and as positive controls (+) the pHL125^{re} and p426-optAraT-S.
   1: pHL125^{re} 2:p426-opt-AraT-S 3:p426HXT7-6HIS 4:pTHStp2
   Medium with 2% D-galactose, 2% D-glucose, 2% mannose, 2% L-arabinose, 0,5% L-arabinose
   All SC medium plates were incubated at 30°C. The L-arabinose plates show growth after 10 days and all other plates after 3 days.

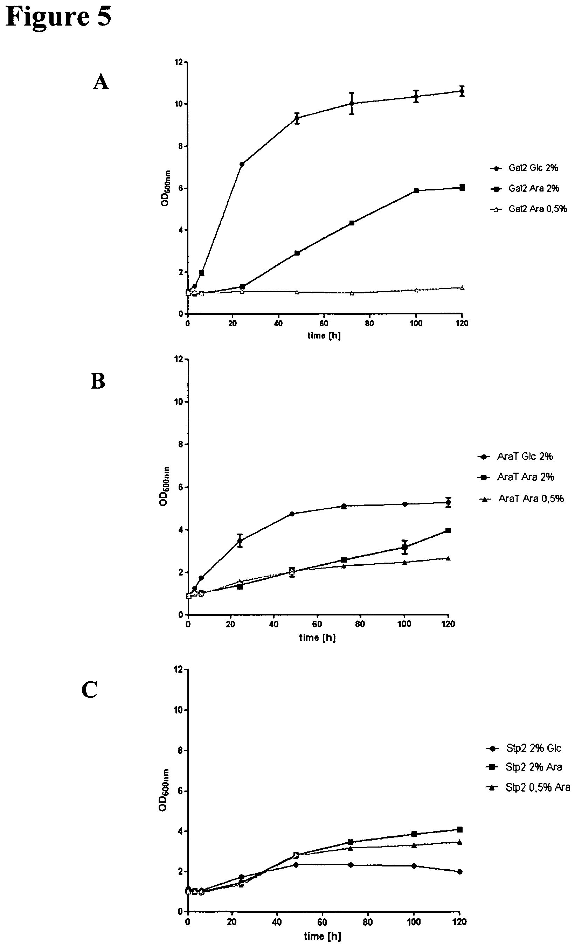

**Figure 5****.** *Growth behaviour on glucose and arabinose with the use of the arabinose transporter.*
   Growth of the MKY06-3P, which additionally also contains the plasmids pHL125^{re} (**A**), p426-opt-AraT-S (**B**) and pTHSTp2 (**C**) in SC medium with 2% glucose, 2% or 0,5% L-arabinose under aerobic conditions. The strains with the various plasmids were adducted in SC medium with the respective carbon source and inoculated with an OD₆₀₀ₙₘ = 1 in 50ml SC medium. The incubation took place in 300 ml shaking flasks under aerobic conditions at 30°C. Samples were taken several times during the 120 hours to determine the optical density.

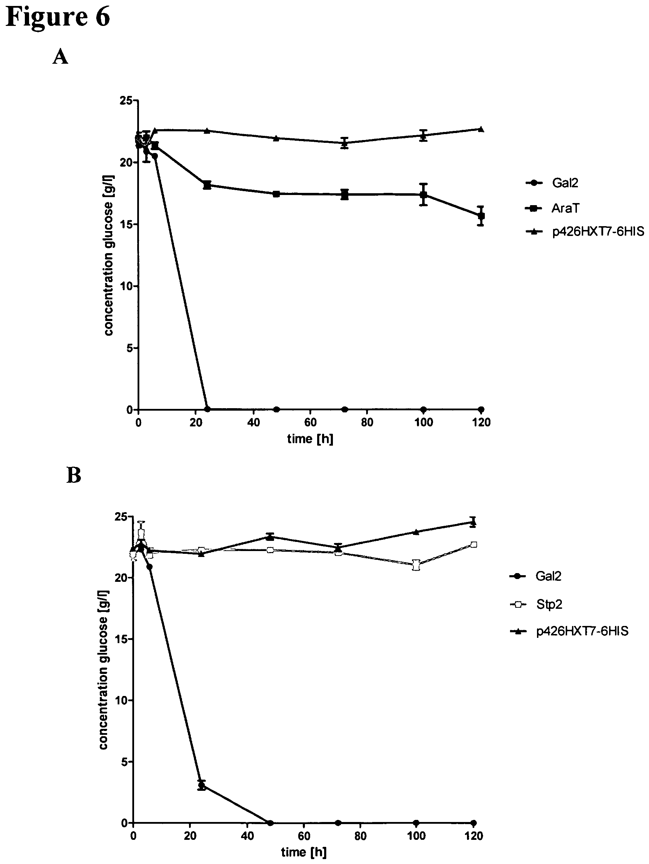

**Figure 6****.** *Comparison of glucose fermentation of AraT and Stp2 on 2% glucose media.* Fermentation analysis of the MKY06-3P containing the plasmids p426-opt-AraT-S (**A**) or pTHSTp2 (**B**) in SC medium with 2% glucose under aerobic conditions. As controls the strain MKY06-3P was used containing the plasmids pHL125^{re} (positive) or p426HXT7-6HIS (negative). The strains with the various plasmids were adducted in SC medium with 2% glucose and inoculated with an OD₆₀₀ₙₘ = 1 in 50ml SC medium. The incubation took place in 300 ml shaking flasks under aerobic conditions at 30°C. Samples were taken several times during the 120 hours to determine the sugar concentration via HPLC analysis.

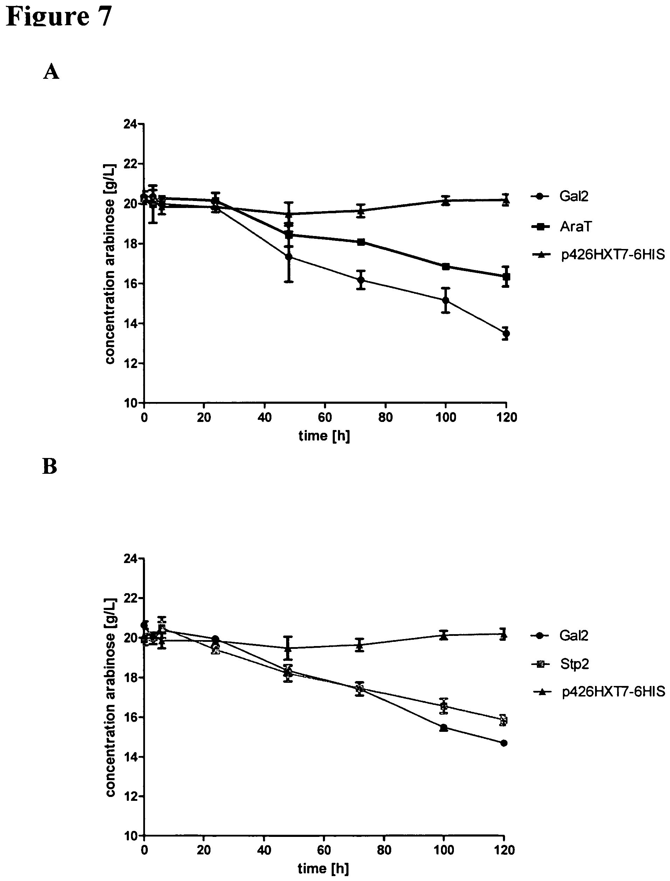

**Figure 7****.** *Comparison of arabinose fermentation of AraT and Stp2 on 2% arabinose media.*
   Fermentation analysis of the MKY06-3P containing the plasmids p426-opt-AraT-S (**A**) or pTHSTp2 (**B**) in SC medium with 2% arabinose under aerobic conditions. As controls the strain MKY06-3P was used containing the plasmids pHL125^{re} (positive) or p426HXT7-6HIS (negative). The strains with the various plasmids were adducted in SC medium with 2% arabinose and inoculated with an OD₆₀₀ₙₘ = 1 in 50ml SC medium. The incubation took place in 300 ml shaking flasks under aerobic conditions at 30°C. Samples were taken several times during the 120 hours to determine the sugar concentration via HPLC analysis.

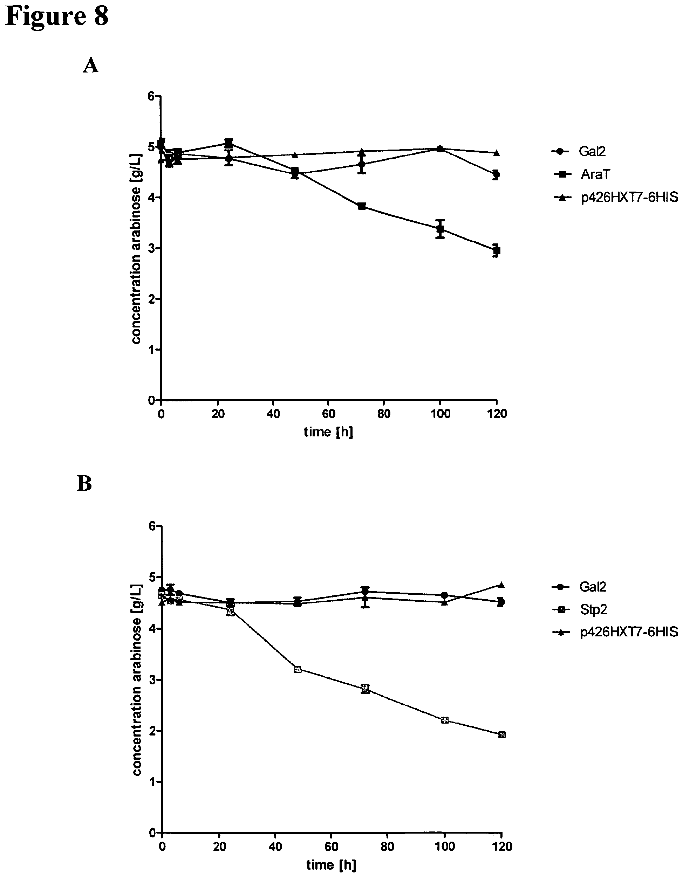

**Figure 8****.** *Comparison of arabinose fermentation of AraT and Stp2 on 0.5% arabinose media.*
   Fermentation analysis of the MKY06-3P containing the plasmids p426-opt-AraT-S (**A**) or pTHSTp2 (**B**) in SC medium with 0.5% arabinose under aerobic conditions. As controls the strain MKY06-3P was used containing the plasmids pHL125^{re} (positive) or p426HXT7-6HIS (negative). The strains with the various plasmids were adducted in SC medium with 0,5% arabinose and inoculated with an OD₆₀₀ₙₘ = 1 in 50ml SC medium. The incubation took place in 300 ml shaking flasks under aerobic conditions at 30°C. Samples were taken several times during the 120 hours to determine the sugar concentration via HPLC analysis.

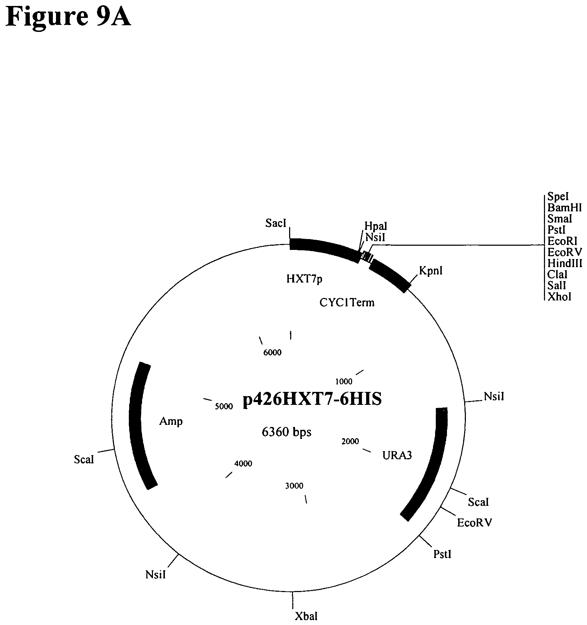

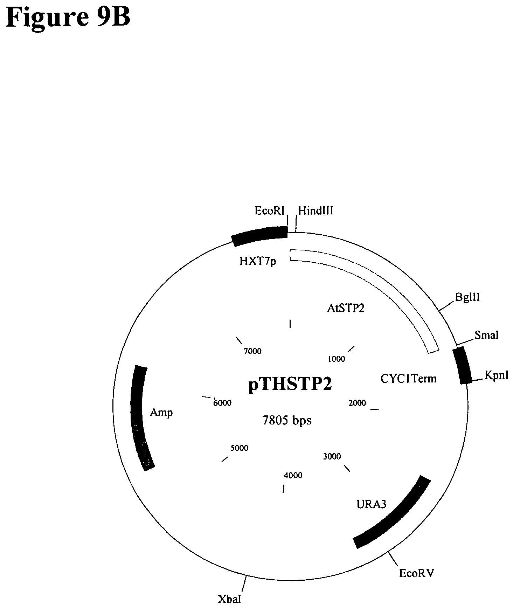

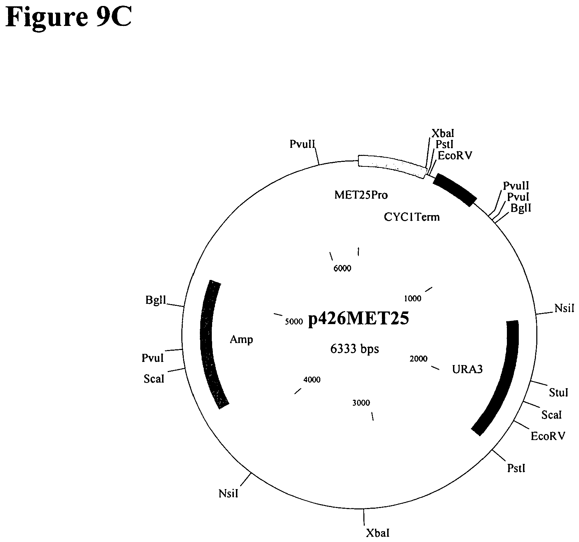

**Figure 9****.** *Vectors used and their structure.*
   The open reading frame (ORF) of the arabinose transporter Stp2 according to the invention was amplified and cloned behind the shortened strong *HXT7* promoter of the plasmid p426HXT7-6HIS (**A**). With this, the plasmid pTHStp2 (**B**) was produced, which has a uracil marker. Another possible expression plasmid is p426Met25 (**C**).

### EXAMPLES

### Methods

### 1. Strains and media

### - Bacteria

### E.coli SURE (Stratagene)

Full medium LB 1% trypton, 0.5% yeast extract, 0.5% NaCl, pH 7.5 (see Sambrook and Russell, 2001)

For selection on a plasmid-coded antibiotic resistance, 40 µg/ml ampicillin was added to the medium after autoclaving. Solid culture media additionally contained 1.9% agar. The culture took place at 37°C.

### - Yeast

### Strain EBY.VW4000

EBY.VW4000 (Genotype: *MATα leu2-3,112ura3-52 trp1-289 his3-Δ1 MAL2-8c SUC2 Δhxt1-17Δgal2 stlΔ::loxP agt1Δ::loxP mph2Δ::loxP mph3Δ::loxP*) (Wieczorke *et al.,* 1999)

### Strain MKY06

MKY06 (Genotype: *MATa leu2-3,112 ura3-52 trp1-289 his3-1 MAL2-8c SUC2 hxt1-17 gal2 stl1::loxP agt1::loxP mph2::loxP mph3::loxP PromTAL1::loxP-Prom-vkHXT7,* description: EBY.VW4000 *PromTAL1::loxP-Prom-vkHXT7*)

### Strain MKY06-3P

MKY06-3P (Genotype: *MATa leu2-3,112 ura3-52 trp-289 his3-1 MAL2-8c SUC2 hxt1-17 gal2 stl1::loxP agt1::loxP mph2::loxP mph3::loxP PromTAL1::loxP-Prom-vkHXT7,* description: EBY.VW4000 *PromTAL1::loxP-Prom-vkHXT*); contains the plasmids p423H7-synthIso, p424H7-synthKin and p425H7-synthEpi.

### - synthetic complete selective medium SC

0.67% yeast nitrogen base w/o amino acids and ammonium sulphate, 0.5% ammonium sulphate, 20mM potassium dihydrogenphosphate, pH 6.3, amino acid/nucleobase solution without the corresponding amino acids for the auxotrophy markers of the plasmids used, carbon source in the respectively indicated concentration

Concentration of the amino acids and nucleobases in the synthetic complete medium (Zimmermann, 1975): adenine (0.08 mM), arginine (0.22 mM), histidine (0.25 mM), isoleucine (0.44 mM), leucine (0.44 mM), lysin (0.35 mM), methionine (0.26 mM), phenylalanine (0.29 mM), threonine (0.48 mM), tryptophan (0.19 mM), tyrosin (0.34 mM), uracil (0.44 mM) and valine (0.49 mM). As carbon sources, L-arabinose, D-glucose, D-galactose, D-mannose and maltose were used.

Solid full and selective media contained in addition 1.9% agar. The culture of the yeast cells took place at 30°C.

### 2. Plasmids

| Plasmid | Source/Reference | Description |
|---|---|---|
| p423H7-synthIso | Wiedemann and Boles, 2008. | Codon-optimised *B.licheniformis araA.* in p423HXT7-6HIS |
| p424H7-synthKin | Wiedemann and Boles, 2008. | Codon-optimised *E.coli araB* in p424HXT7-6HIS, mutation in araB |
| p425H7-synthEpi | Wiedemann and Boles, 2008. | Codon-optimised *E.coli araD* in p425HXT7-6HIS |
| p426HXT7-6HIS | Becker and Boles, 2003 | 2µ plasmid for over-6HIS expression of genes and for production of fusion proteins with 6xHis-Epitope; *URA3* marker gene, shortened *HXT7* |

| | | |
|---|---|---|
| | | promoter and *CYC1* terminator |
| pHL125^{re} | Liang and Gaber, 1996 | 2µ plasmid with the *GAL2* gene expressed behind the *ADH1* promoter, *URA3* marker gene, reisolated from JBY25-4M |
| p426H7-araT | WO 2008/080505 | 2µ plasmid with *P. stipitis* AraT behind the shortened *HXT7* promoter, *URA3* marker gene |
| pTHStp2 | Hamacher et al., 2002 | 2µ plasmid expressed with the *Arabidopis thaliana STP2* behind the shortened *HXT7* promoter, *URA3* marker gene |

### 3. Transformation

### - Transformation of S.cerevisiae

The transformation of *S.cerevisiae* was carried out by the lithium-acetate method (Gietz and Woods, 2002).

### - Transformation of E.coli

The transformation of the *E.coli* cells took place by the electroporation method (Dower *et al.*, 1988; Sambrook and Russell, 2001) by means of an Easyject prima apparatus from EQUIBO.

### 4. Preparation of DNA

### - Isolation of plasmid-DNA from S.cerevisiae

The cells of a stationary yeast culture (5ml) were harvested, washed and re-suspended in 100 µl buffer 1 (taken from the "Plasmid Mini Kit"). After the addidion of 200 µl buffer 2 and 2/3 volume glass beads (diameter = 0.45 mm), the cells were solubilised for 8 min on a Vibrax (Janke and Kunkel, Vibrax-VXR) at 4°C. The supernatant was mixed with 150 µl buffer 3 and incubated for 10 min on ice. After centrifuging for 15 minutes at 10000 R/min, the supernatant was used and the plasmid-DNA was precipitated with 400 µl isopropanol (-20°C, 10 min). The DNA, which was pelleted through centrifuging (30 min, 13000 rpm) was washed with 70% cold ethanol and held in 20 µl water. The DNA was then used for a transformation in *E.coli* or a DNA amplification by means of PCR.

### - Isolation of plasmid-DNA from E. coli

The isolation of plasmid-DNA from *E.coli* took place with the "Plasmid Mini Kit" of the company Qiagen, according to the manufacturer's information.

### - Determining the DNA concentration

The DNA concentration is measured by spectral photometry in a wavelength range of 240-300 nm. If the purity of the DNA, determined by the quotient E260nm/E280nm is 1.8, then the extinction E260nm = 1.0 corresponds to a DNA concentration of 50 µg dsDNA/ml (Sambrook and Russell, 2001).

### 5. DNA amplification by means of PCR

### - Use of the Expand™ High Fidelity System

The polymerase chain reaction (PCR) took place with the "Expand™ High Fidelity PCR System" of the company Roche, according to the manufacturer's information. 0.2 mM dNTP-mix, 1x buffer 2 (contains 1.5 mM MgC12), 1 U polymerase and 100 pmol each of the corresponding oligonucleotide primers were added together to the plasmid- or genomic DNA to be amplified. The PCR reaction was carried out in a thermocycler (Techne) or mastercycler (Eppendorf).

For the amplification of the DNA, the following temperature cycles were selected.
1. 1x 95°C, 4min denaturing of the DNA
2. 18-30x 95°C, 30 sec denaturing of the DNA
   55°C, 30 sec binding of the primers to the DNA (annealing)
   72°C, 2 min DNA synthesis (elongation)
3. 1x 72°C, 4 min synthesis (elongation)

The number of synthesis steps, the annealing temperature and the elongation time were adapted to the specific melting temperatures of the oligonucleotides which were used or to the size of the product which was to be expected. The PCR products were checked by a subsequent agarose gel electrophoresis and then purified.

### - DNA purification of PCR products

The purification of the PCR products took place with the "QIAquick PCR Purification Kit" of the company Qiagen, according to the manufacturer's information.

### - Gel electrophoretic separation of DNA fragments

The separation of DNA fragments with a size of 0.15-20 kb took place in 1-4% agarose gels. 1xTAE buffer (40 mM Tris, 40mM acetic acid, 2 mM EDTA) was used as gel- and running buffer (Sambrook and Russell, 2001). Serving as marker was either a lambda phage DNA cut with the restriction endonucleases *Eco*RI and *Hind*III, or the 2-log DNA ladder (NEB). Before application, the DNA samples were mixed with 1/10 volume blue marker (1xTAE buffer, 10% glycerine, 0.004% bromophenol blue). After the separation, the gels were incubated in an ethidium bromide bath and the DNA fragments were made visible by irradiation with UV light (254 nm).

### - Isolation of DNA fragments from agarose gels

The desired DNA fragment was cut out from the TAE agarose gel under longwave UV light (366 nm) and isolated with the "QIAex II Gel Extraction Kit" or the "QIA-quick Gel Extraction Kit" of the company Qiagen, according to the manufacturer's information.

### 6. Enzymatic modification of DNA

### - DNA restriction

Sequence-specific splittings of the DNA with restriction endonucleases were carried out under the incubation conditions recommended by the manufacturer for 2-3 hours with 2-5U enzyme per µg DNA.

### 7. HPLC analyses

The samples taken in the tests were centrifuged for 10 min at 3000 R/min, in order to pellet the yeast cells. The supernatant was removed and immediately frozen at -20°C. For the protein precipitation, subsequently 50% sulphosalicylic acid was added, mixed, and centrifuged off for 30 min at 13000 R/min and 4°C. The supernatant was removed, a 1/10 dilution with water was produced therefrom and used for the HPLC analyses. Serving as standards for the measurements were samples with D-glucose, L-arabinose, glycerol, acetate and ethanol, which were used in concentrations of 0.01%w/w, 0.05%w/w, 0.1%w/w, 0.5%w/w and 1.0%w/w. The sugar concentrations were measured by means of BioLC (Dionex). The autosampler "AS50", the column oven "TCC-100", the gradient pump "GS50" (all Dionex) and the RI detector "RI-101" (Shodex) were used in the measurement. As a column, the VA 300/7.7 nucleogel sugar 810H (Machery-Nagel) was used with 20% sulphuric acid as eluent (0.6ml/min). For the evaluation of the analysis data, the Chromeleon™ program (Version 6.50, Dionex) was used.

### Example 1 Structure of a test system for the examination of arabinose transporters

### A) Construction of the MKY06

In the yeast strain EBY.VW4000 all the genes of the hexose transporter family and in addition three genes of the maltose transporter family were deleted. This strain grew on maltose medium unchanged, but was no longer able to grow on glucose, fructose and mannose and only very weakly on galactose (Wieczorke *et al., 1999*). As all hexose transporters are deleted, it can be assumed that the strain also can no longer receive any pentoses and is therefore suitable for pentose transport investigations.

In preceding tests (see Becker and Boles, 2003), it had been found that in addition to a functional L-arabinose metabolic pathway, also an increased activity of transaldolase was necessary for the use of L-arabinose. For this reason, by exchange of the endogenous promoter of *TAL1* in EBY.VW4000 for the shortened *HXT7* promoter *TAL1* was over-expressed. This strain was named MKY06 and is provided, with the plasmids for the L-arabinose metabolism and a transporter which can transport L-arabinose, to grow on this carbon source.

### B) Introduction of the L-arabinose metabolic pathway

The strain MKY06 was transformed with the plasmids p423H7-synthIso, p424H7-synthKin and p425H7-synthEpi, so that it obtains the capability of L-arabinose use. The transformation with the three plasmids took place simultaneously. The transformants were plated on SC medium with 2% maltose. In a further transformation, as positive control in addition the transporter Gal2, known as L-arabinose transporter, was transformed in and as negative control the empty plasmid p426HXT7-6HIS and plated again on medium plates containing maltose. The positive control, which contains an L-arabinose transporter and the three plasmids for the L-arabinose use and over-expresses transaldolase, should be able to grow on L-arabinose. The negative control should show no growth owing to the absent transporter. This was investigated.

### C) Checking the test system

In order to be able to use the constructed test system further, firstly the positive and negative controls had to be investigated with regard to their growth. Several colonies of the transformants obtained on the SC plates with 2% maltose were removed with a sterile inoculating loop and smeared on SC plates with 2% L-arabinose and incubated at 30°C for ten days. After this time, the positive controls showed a distinct growth and the negative control, as expected, showed no growth.

This test system was therefore functional and was able to be used for the investigation of L-arabinose transporters. The positive and negative control mentioned here always served as a comparison in this.

### Example 2 Growth behaviour of pTHStp2

The test system was now used in order to test pTHStp2 for growth on medium with different sugar sources.

### A) Carrying out the screen

The plasmid pTHStp2 which was constructed and described by Hamacher et al. 2002 was transformed into the strain MKY06-3P and smeared on SC medium with 2% maltose. The colonies obtained after three days at 30°C were replica-plated on SC medium plates with different carbon sources, in order to test the substrate spectrum.

Growth was only possible when the AtStp2 transporter was able to transport the particular carbon source.

The results are shown in Figures 4. The strain MKY06-3P with the pTHStp2 plasmid grows only on plates containing galactose or arabinose and less on mannose. There was no growth on media containing glucose.

In order to be able to rule out genomic mutations which could be responsible for the growth, the colonies which were found were smeared on complete medium with 5-FOA. Thereby, selection was carried out on a loss of the plasmid of the gene bank. With renewed smearing on L-arabinose medium, these colonies of the 5-FOA plate were no longer able to grow hereon. Therefore, a genomic mutation was able to be ruled out as the cause of the growth.

### Example 3 Characterization of the arabinose transporter (AtStp2)

### A) Sequencing

The open reading frame of the AtStp2 in the plasmid pTHStp2 was sequenced with overlapping regions. The sequencing shows that there were no mutations in the open reading frame.

### B) Examples for vectors for AtStp2

The open reading frame (ORF) of AtStp2 was amplified and cloned behind the shortened strong *HXT7* promoter of the plasmid p426HXT7-6HIS. With this, the plasmid pTHStp2 was produced, which has a uracil marker.

Another possible expression plasmid is p426Met25. For vector maps, see Figures 9A to 9C.

Further possible expression vectors are pYES260, pYES263, pVTU260, pVTU263, pVTL260 and pVTL263. Further information on these vectors is to be found at http://web.uni-frankfurt.de/fb15/mikro/euroscarf/data/km_expr.html.

### C) Growth behaviour and sugar consumption

The growth of the strain MKY06-3P + pTHStp2 was investigated under aerobic conditions as a function of the L-arabinose concentration and on glucose in the medium. As controls, strains derived from MKY06-3P were used, which additionally also contained the plasmid pHL125^{re}, p426HXT7-6HIS or p426-opt-AraT-S. p426-opt-AraT-S was derived from plasmid p426H7-araT which contains araT, the arabinose transporter of *Pichia stipitis* as published in WO 2008/080505, by replacing the coding sequence of AraT from *Pichia stipitis* with a codon-optimised sequence of AraT adapted to the glycolytic codon usage of *S. cerevisiae* (Wiedemann and Boles 2008).

The strains with the plasmids coding for transporters were adducted in SC medium with 2% L-arabinose and inoculated with an OD₆₀₀ₙₘ = 1 in 50 ml SC medium with 0.5%, or 2% L-arabinose, or 2% glucose. The strain containing the empty vector was adducted in SC medium with 1% maltose. The incubation took place in 300ml shaking flasks under aerobic conditions at 30°C. Samples were taken several times in the day to determine the optical density and the carbon source concentration.

The results are shown in Figures 5 to 8. The strain MKY06-3P + pTHStp2 reached a higher optical density on arabinose 2% and 0.5% than on glucose 2% (Figure 5C). The other strains containing the pHL125^{re} or p426-opt-AraT-S grew more quickly on glucose media than on arabinose media (Figure 5A & 5B).

With an L-arabinose concentration of 2% the strains containing transporters grew more or less similar in the first hours, however, pHL125^{re} reaches a higher optical density at the end of growth with this concentration.

With 0.5% L-arabinose a distinct advantage of pTHStp2 and p426-opt-AraT-S is shown compared with pHL125^{re} (Figure 5B & 5C).

These findings were confirmed by the analysis of the sugar consumption (Figures 6 to 8). All strains containing arabinose transporters fermented arabinose but only MKY06-3P + pTHStp2 and MKY06-3P + p426-opt-AraT-S consumed arabinose at a concentration of 0.5% arabinose showing that these transporters have a higher affinity to arabinose than Gal2.

While MKY06-3P + pHL125^{re} as well as MKY06-3P + p426-opt-AraT-S fermented glucose, MKY06-3P + pTHStp2 consumed no glucose indicating that this transporter is a specific arabinose transporter which transports arabinose but no glucose.

It is therefore shown that the L-arabinose uptake system according to the invention makes it possible for the recombinant *S.cerevisiae* cells to use L-arabinose substantially more efficiently.

### REFERENCES

Becker, J. and Boles, E. (2003) A modified Saccharomyces cerevisiae strain that consumes L-Arabinose and produces ethanol. Appl Environ Microbiol 69(7),4144-50*.*
Boles, E. (2002) in "Transmembrane Transporters", Editor Michael W.Quick, WILEY-LISS, 19-36.
Dien, B.S., Kurtzman, C.P., Saha, B.C. and Bothast, R.J. (1996) Screening for L-arabinose fermenting yeasts. Appl Biochem Biotechnol 57-58, 233-42.
Dower, W.J., Miller, J.F. and Ragsdale, C.W. (1988) High efficiency transformation of E.coli by high voltage electroporation. Nucleic Acids Res 16(13), 6127-45.
Gietz, R.D. and Woods, R.A. (2002) Transformation of yeast by lithium acetate/single-stranded carrier DNA/polyethylene glycol method. Methods Enzymol 350,87-96.
Hahn-Hagerdal, B., Wahlbom, C.F., Gardonyi, M., van Zyl, W.H., Cordero Otero, R.R. and Jonsson, L.J. (2001) Metabolic engineering of Saccharomyces cerevisiae for xylose utilization. Adv Biochem Eng Biotechnol 73,53-84.
Hamacher T., Becker J., Gárdonyi M., Hahn-Hägerdal B., Boles E. (2002) Characterization of the xylose-transporting properties of yeast hexose transporters and their influence on xylose utilization. Microbiology 148 (9):2783-8
Jeppson M, Bengtsson O, Franke K, Lee H, Hahn-Hagerdal B, Gorwa-Grauslund MF. (2006) The expression of a Pichia stipitis xylose reductase mutant with higher K(M) for NADPH increases ethanol production from xylose in recombinant Saccharomyces cerevisiae. Biotechnol Bioeng. 93(4):665-73.
Jin YS, Jeffries TW. (2004) Stoichiometric network constraints on xylose metabolism by recombinant Saccharomyces cerevisiae. Metab Eng. 6(3):229-38.
Katahira S, Mizuike A, Fukuda H, Kondo A. (2006) Ethanol fermentation from lignocellulosic hydrolysate by a recombinant xylose- and cellooligosaccharide-assimiliating yeast strain. Appl Microbiol Biotechnol. 72(6):1136-43.
Kotter,P. and Ciriacy, M. (1993) Xylose fermentation by Sacharomyces cerevisiae. Appl Microbiol Biotechnol 38, 776-783.
Kou, S.C., Christensen, M.S. and Cirillo, V.P. (1970) Galactose transport in Saccharomyces cerevisiae. II. Characteristics of galactose uptake and exchange in galactokinaseless cells. J Bacteriol 103(3), 671-8.
Kuyper, M., Toirkens, M.J., Diderich, J.A., Winkler, A.A., van Dijken, J.P. and Pronk, J.T. (2005b) Evolutionary engineering of mixed-sugar utilization by a xylose-fermenting Saccharomyces cerevisiae strain. FEMS Yeast Res 5(10), 925-34.
Kuyper, M., Winkler, A.A., van Dijken, J.P. and Pronk, J.T. (2004) Minimal metabolic engineering of Saccharomyces cerevisiae for efficient anaerobic xylose fermentation: a proof of principle. FEMS Yeast Res 4(6), 655-64.
Liang, H. and Gaber, R.F. (1996) A novel signal transduction pathway in Saccharomyces cerevisiae defined by Snf3-regulated expression of HXT6. Mol Biol Cell 7(12):1953-66.
Lucas, C. and Uden, N.v. (1986) Transport of hemicellulose monomers in the xylose-fermenting yeast Candida shehatae. Appl Microbiol Biotechnol 23,491-495.
Sambrook J. and Russell D.W. (2001) Molecular cloning. A laboratory manual. New York: Cold Spring Harbor
Pitkanen JP, Rintala E, Aristidou A, Ruohonen L, Penttila M. (2005) Xylose chemostat isolates of Saccharomyces cerevisiae show altered metabolite and enzyme levels compared with xylose, glucose, and ethanol metabolism of the original strain. Appl Microbiol Biotechnol. 67(6):827-37.
Richard, P., Putkonen, M., Vaananen, R., Londesborough, J. and Penttila, M. (2002) The missing link in the fungal L-arabinose catabolic pathway, identification of the L-xylulose reductase gene. Biochemistry 41 (20), 6432-7.
Truernit E., Stadler R., Baier K., Sauer N. (1999) A male gametophyte-specific monosaccharide transporter in Arabidopsis. Plant J. 17 (2):191-201.
Verho R, Penttilä M, Richard P. Cloning of Two Genes (LAT1,2) Encoding Specific L: - Arabinose Transporters of the L: -Arabinose Fermenting Yeast Ambrosiozyma monospora. Appl Biochem Biotechnol. 2011 Jan 21. [Epub ahead of print] DOI 10.1007/s12010-011-9161-y
Wieczorke, R., Krampe, S., Weierstall, T., Freidel, K., Hollenberg, C.P. and Boles, E. (1999) Concurrent knock-out of at least 20 transporter genes is required to block uptake of hexoses in Saccharaomyces cerevisiae. FEBS Lett 464(3), 123-8.
Wiedemann B., Boles E. (2008) Codon-optimized bacterial genes improve L-Arabinose fermentation in recombinant Saccharomyces cerevisiae. Appl Environ Microbiol. 74 (7):2043-50
Zaldivar, J., Nielsen, J. and Olsson, L. (2001) Fuel ethanol production from lignocellulose: a challenge for metabolic engineering and process integration. Appl Microbiol Biotechnol 56(1-2), 17-34.
Zimmermann, F.K. (1975) Procedures used in the induction of mitotic recombination and mutation in the yeast Saccharomyces cerevisiae. Mutat Res 31 (2), 71-86.

## Claims

1. Use of a nucleic acid molecule comprising a nucleic acid sequence, which codes for a plant pentose transporter, for the transformation of a cell, wherein the transformation enables the cell to take up L-arabinose,
wherein the cell is a yeast cell,
and wherein the plant pentose transporter comprises an amino acid sequence, which is at least 70%, preferably at least 80%, more preferably at least 90% identical to the amino acid sequence of SEQ ID NO: 1 and has an *in vitro* and/or *in vivo* pentose transport function.

2. The use according to claim 1, wherein the plant pentose transporter *in vitro* and/or *in vivo* transports L-arabinose and does not transport D-glucose.

3. The use according to claim 1 or 2, wherein the plant pentose transporter has an amino acid sequence of SEQ ID NO:1.

4. The use according to any of claims 1 to 3, wherein the nucleic acid sequence coding for a plant pentose transporter comprises a nucleic acid sequence, which is at least 70% identical, preferably 80%, more preferably 90% identical to the nucleic acid sequence of SEQ ID NO: 2.

5. The use according to any of the preceding claims, wherein the nucleic acid molecule is a nucleic acid expression construct, which comprises regulatory sequences being operatively linked with the nucleic acid sequence coding for the plant pentose transporter,
and/or wherein the nucleic acid expression construct further comprises 5' and/or 3' recognition sequences and/or selection markers.

6. The use according to any of the preceding claims, wherein the yeast cell is a member of a genus selected from the group of *Saccharomyces, Kluyveromyces, Candida, Pichia, Schizosaccharomyces, Hansenula, Kloeckera, Schwanniomyces, Arxula* and *Yarrowia* and preferably a member of a species selected from the group of *S. cerevisiae, S. bulderi, S. barnetti, S. exiguus, S. uvarum, S. diastaticus, K. lactis, K. marxianus, K. fragilis, H* .*polymorpha, P.pastoris* and *Y. lipolytica.*

7. The use according to any of the preceding claims, wherein the cell further contains nucleic acid sequences coding for proteins of an arabinose metabolic pathway.

8. The use according to any of the preceding claims for
- the conversion and metabolization, particularly recombinant fermentation, of biomaterial containing L-arabinose,
- the production of bio-based chemicals or biofuels,
- the production of bioalcohols, preferably bioethanol and/or biobutanol.

9. A yeast cell, which is transformed with a nucleic acid expression construct comprising:
(a) a nucleic acid sequence coding for a plant pentose transporter,
wherein the plant pentose transporter comprises an amino acid sequence, which is at least 70%, preferably at least 80%, more preferably at least 90% identical to the amino acid sequence of SEQ ID NO: 1 and has an *in vitro* and/or *in vivo* pentose transport function,
(b) regulatory elements operatively linked with the nucleic acid sequence, allowing for the expression of the plant pentose transporter in the yeast cell,
wherein the expression of the nucleic acid expression construct imparts to the yeast cell the capability to take up L-arabinose,
and wherein the yeast cell over-expresses a TAL1 (transaldolase) gene and expresses the genes araA (L-arabinose-isomerase), araB (L-ribulokinase) and araD (L-ribulose-5-P-4-epimerase).

10. The yeast cell according to claim 10, wherein the yeast cell is transformed with a nucleic acid molecule as defined in any of claims 1 to 5,
and/or wherein the yeast cell is as defined in claim 6 or 7.

11. The yeast cell according to any of claims 9 or 10, furthermore expressing one or more enzymes, which impart to the cell the capability to produce one or more further metabolization products, the further metabolization products prefereably being selected from bio-based chemicals or biofuels.

12. Use of a yeast cell according to any of claims 9 to 11 for
- the conversion and metabolization, particularly recombinant fermentation, of biomaterial containing L-arabinose,
- the production of bio-based chemicals or biofuels,
- the production of bioalcohols, preferably bioethanol and/or biobutanol.

13. A method for the production of bioalcohol comprising the steps of:
(a) converting a medium containing an L-arabinose source with a cell according to any of claims 9 to 11, which converts L-arabinose to a bioalcohol,
(b) optionally obtaining the bioalcohol,
wherein the bioalcohol is bioethanol and/or biobutanol.

14. A method for the production of a metabolization product comprising the steps of:
(a) converting a medium containing an L-arabinose source with a cell according to claim 11, which converts L-arabinose to produce the metabolization product,
(b) optionally obtaining the metabolization product,
the metabolization product preferably being selected from the group of bio-based chemicals or biofuels, such as lactic acid, acetic acid, succinic acid, malic acid, 1-butanol, isobutanol, 2-butanol, other alcohols, amino acids, 1,3-propanediol, ethylene, glycerol, a β-lactam antibiotic or a cephalosporin, alkanes, terpenes, isoprenoids or the precursor molecule amorphadiene of the antimalarial drug artemisinin.

15. The method according to claim 13 or 14, wherein the medium contains a further carbon source, particularly hexose, more particularly glucose.

## Patentansprüche

1. Verwendung eines Nukleinsäuremoleküls, umfassend eine Nukleinsäuresequenz, die für einen Pflanzen-Pentosetransporter kodiert, zur Transformation einer Zelle, wobei die Transformation die Zelle in die Lage versetzt, L-Arabinose aufzunehmen,
wobei die Zelle eine Hefezelle ist,
und wobei der Pflanzen-Pentosetransporter eine Aminosäuresequenz umfasst, die wenigstens 70%, bevorzugt wenigstens 80%, bevorzugter wenigstens 90% identisch mit der Aminosäuresequenz von SEQ ID NO: 1 ist und eine *in vitro-* und/oder *in vivo-*Pentosetransportfunktion hat.

2. Verwendung nach Anspruch 1, wobei der Pflanzen-Pentosetransporter L-Arabinose *in vitro* und/oder *in vivo* transportiert und nicht D-Glucose transportiert.

3. Verwendung nach Anspruch 1 oder 2, wobei der Pflanzen-Pentosetransporter eine Aminosäuresequenz von SEQ ID NO:1 hat.

4. Verwendung nach einem der Ansprüche 1 bis 3, wobei die Nukleinsäuresequenz, die für einen Pflanzen-Pentosetransporter kodiert, eine Nukleinsäuresequenz umfasst, die wenigstens 70% identisch, bevorzugt 80%, bevorzugter 90% identisch mit der Nukleinsäuresequenz von SEQ ID NO: 2 ist.

5. Verwendung nach einem der vorangehenden Ansprüche, wobei das Nukleinsäuremolekül ein Nukleinsäureexpressionskonstrukt ist, welches regulatorische Sequenzen umfasst, die mit der Nukleinsäuresequenz, die für den Pflanzen-Pentosetransporter kodiert, operativ verknüpft sind,
und/oder wobei das Nukleinsäureexpressionskonstrukt weiterhin 5'- und/oder 3'-Erkennungssequenzen und/oder Selektionsmarker umfasst.

6. Verwendung nach einem der vorangehenden Ansprüche, wobei die Hefezelle ein Mitglied eines Genus ist, ausgewählt aus der Gruppe von *Saccharomyces, Kluyveromyces, Candida, Pichia, Schizosaccharomyces, Hansenula, Kloeckera, Schwanniomyces*, *Arxula* und *Yarrowia* und bevorzugt ein Mitglied einer Spezies ist, ausgewählt aus der Gruppe von *S*. *cerevisiae, S. bulderi, S. barnetti, S. exiguus, S. uvarum, S. diastaticus, K. lactis, K. marxianus, K. fragilis, H. polymorpha, P. pastoris* und *Y. lipolytica.*

7. Verwendung nach einem der vorangehenden Ansprüche, wobei die Zelle weiterhin Nukleinsäuresequenzen enthält, die für Proteine eines metabolischen Arabinosewegs kodieren.

8. Verwendung nach einem der vorangehenden Ansprüche, zur
- Umwandlung und Metabolisierung, insbesondere rekombinanten Fermentation, von Biomaterial, enthaltend L-Arabinose,
- Produktion von bio-basierten Chemikalien oder Biotreibstoffen,
- Produktion von Bioalkoholen, bevorzugt Bioethanol und/oder Biobutanol.

9. Hefezelle, die mit einem Nukleinsäureexpressionskonstrukt transformiert ist, umfassend:
(a) eine Nukleinsäuresequenz, die für einen Pflanzen-Pentosetransporter kodiert,
wobei der Pflanzen-Pentosetransporter eine Aminosäuresequenz umfasst, die wengistens 70%, bevorzugt wenigstens 80%, bevorzugter wenigstens 90% identisch mit der Aminosäuresequenz von SEQ ID NO: 1 ist und eine *in vitro-* und/oder *in vivo-*Pentosetransportfunktion hat,
(b) regulatorische Elemente, die mit der Nukleinsäuresequenz operativ verknüpft sind, und die Expression des Pflanzen-Pentosetransporters in der Hefezelle ermöglichen,
wobei die Expression des Nukleinsäureexpressionskonstrukts der Hefezelle die Fähigkeit verleiht, L-Arabinose aufzunehmen,
und wobei die Hefezelle ein TAL1 (Transaldolase)-Gen überexprimiert und die Gene araA (L-Arabinose-Isomerase), araB (L-Ribulokinase) und araD (L-Ribulose-5-P-4-Epimerase).

10. Hefezelle nach Anspruch 10, wobei die Hefezelle mit einem Nukleinsäuremolekül transformiert ist, wie in einem der Ansprüche 1 bis 5 definiert,
und/oder wobei die Hefezelle wie in Anspruch 6 oder 7 definiert ist.

11. Hefezelle nach einem der Ansprüche 9 oder 10, weiterhin ein oder mehrere Enzyme exprimierend, die der Zelle die Fähigkeit verleihen, ein oder mehrere Metabolisierungsprodukte zu erzeugen, wobei die weiteren Metabolisierungsprodukte bevorzugt ausgewählt sind aus bio-basierten Chemikalien oder Biotreibstoffen.

12. Verwendung einer Hefezelle nach einem der Ansprüche 9 bis 11 zur
- Umwandlung und Metabolisierung, insbesondere rekombinanten Fermentation, von Biomaterial, enthaltend L-Arabinose,
- Produktion von bio-basierten Chemikalien oder Biotreibstoffen,
- Produktion von Bioalkoholen, bevorzugt Bioethanol und/oder Biobutanol.

13. Verfahren zur Herstellung von Bioalkohol, umfassend die Schritte:
(a) Umwandeln eines Mediums, enthaltend eine L-Arabinosequelle, mit einer Zelle gemäß einem der Ansprüche 9 bis 11, welche L-Arabinose in einen Bioalkohol umwandelt,
(b) fakultatives Erhalten des Bioalkohols,
wobei der Bioalkohol Bioethanol und/oder Biobutanol ist.

14. Verfahren zur Herstellung eines Metabolisierungsprodukts, umfassend die Schritte:
(a) Umwandeln eines Mediums, enthaltend eine L-Arabinosequelle mit einer Zelle nach Anspruch 11, die L-Arabinose umwandelt, um das Metabolisierungsprodukt zu erzeugen,
(b) fakultatives Erhalten des Metabolisierungsprodukts,
wobei das Metabolisierungsprodukt bevorzugt ausgewählt ist aus der Gruppe der bio-basierten Chemikalien oder Biotreibstoffe, wie etwa Milchsäure, Essigsäure, Bernsteinsäure, Äpfelsäure, 1-Butanol, Isobutanol, 2-Butanol, anderen Alkoholen, Aminosäuren, 1,3-Propandiol, Ethylen, Glycerol, ein β-Lactam-Antibiotikum oder ein Cephalosporin, Alkanen, Terpenen, Isoprenoiden oder dem Vorläufermolekül Amorphadien der Antimalaria-Arznei Artemisinin.

15. Verfahren nach Anspruch 13 oder 14, wobei das Medium weiterhin eine Kohlenstoffquelle enthält, insbesondere Hexose, weiterhin insbesondere Glucose.

## Revendications

1. Utilisation d'une molécule d'acide nucléique comprenant une séquence d'acide nucléique, qui code pour un transporteur de pentose de plante, pour la transformation d'une cellule, dans laquelle la transformation permet à la cellule d'absorber le L-arabinose,
dans laquelle la cellule est une cellule de levure,
et dans laquelle le transporteur de pentose de plante comprend une séquence d'acides aminés, qui est identique à au moins 70 %, de préférence à au moins 80 %, de manière davantage préférée à au moins 90 % à la séquence d'acides aminés de SEQ ID NO : 1 et qui possède une fonction de transport de pentose *in vitro* et/ou *in vivo.*

2. Utilisation selon la revendication 1, dans laquelle le transporteur de pentose de plante transporte *in vitro* et/ou *in vivo* le L-arabinose et ne transporte pas le D-glucose.

3. Utilisation selon la revendication 1 ou 2, dans laquelle le transporteur de pentose de plante possède une séquence d'acides aminés de SEQ ID NO : 1.

4. Utilisation selon l'une quelconque des revendications 1 à 3, dans laquelle la séquence d'acide nucléique codant pour un transporteur de pentose de plante comprend une séquence d'acide nucléique, qui est identique à au moins 70 %, de préférence à 80 %, de manière davantage préférée à 90 % à la séquence d'acide nucléique de SEQ ID NO : 2.

5. Utilisation selon l'une quelconque des revendications précédentes, dans laquelle la molécule d'acide nucléique est une construction d'expression d'acide nucléique, qui comprend des séquences de régulation liées de manière fonctionnelle à la séquence d'acide nucléique codant pour le transporteur de pentose de plante,
et/ou dans laquelle la construction d'expression d'acide nucléique comprend en outre des séquences de reconnaissance en 5' et/ou 3' et/ou des marqueurs de sélection.

6. Utilisation selon l'une quelconque des revendications précédentes, dans laquelle la cellule de levure est un membre d'un genre choisi dans le groupe comprenant *Saccharomyces, Kluyveromyces, Candida, Pichia, Schizosaccharomyces, Hansenula, Kloeckera, Schwanniomyces, Arxula* et *Yarrowia* et de préférence un membre d'une espèce choisie dans le groupe comprenant *S. cerevisiae, S. bulderi, S. barnetti, S. exiguus, S. uvarum, S. diastaticus, K. lactis, K. marxianus, K. fra gilis, H. polymorpha, P. pastoris* et *Y. lipolytica.*

7. Utilisation selon l'une quelconque des revendications précédentes, dans laquelle la cellule contient en outre des séquences d'acide nucléique codant pour des protéines d'une voie métabolique de l'arabinose.

8. Utilisation selon l'une quelconque des revendications précédentes pour
- la conversion et la métabolisation, en particulier la fermentation recombinante, d'un biomatériau contenant du L-arabinose,
- la production de produits chimiques ou de biocarburants biosourcés,
- la production de bioalcools, de préférence de bioéthanol et/ou de biobutanol.

9. Cellule de levure, qui est transformée avec une construction d'expression d'acide nucléique comprenant :
(a) une séquence d'acide nucléique codant pour un transporteur de pentose de plante,
dans laquelle le transporteur de pentose de plante comprend une séquence d'acides aminés, qui est identique à au moins 70 %, de préférence à au moins 80 %, de manière davantage préférée à au moins 90 % à la séquence d'acides aminés de SEQ ID NO : 1 et qui possède une fonction de transport de pentose *in vitro* et/ou *in vivo,*
(b) des éléments de régulation liés de manière fonctionnelle à la séquence d'acide nucléique, pour permettre l'expression du transporteur de pentose de plante dans la cellule de levure,
dans laquelle l'expression de la construction d'expression d'acide nucléique confère à la cellule de levure la capacité d'absorber le L-arabinose,
et dans laquelle la cellule de levure surexprime un gène TAL1 (transaldolase) et exprime les gènes araA (L-arabinose-isomérase), araB (L-ribulokinase) et araD (L-ribulose-5-P-4-épimérase).

10. Cellule de levure selon la revendication 10, dans laquelle la cellule de levure est transformée avec une molécule d'acide nucléique telle que définie dans l'une quelconque des revendications 1 à 5,
et/ou dans laquelle la cellule de levure est telle que définie dans la revendication 6 ou 7.

11. Cellule de levure selon l'une quelconque des revendications 9 ou 10, exprimant en outre une ou plusieurs enzymes, qui confèrent à la cellule la capacité de produire un ou plusieurs produits de métabolisation supplémentaires, les produits de métabolisation supplémentaires étant choisis de préférence parmi des produits chimiques ou des biocarburants biosourcés.

12. Utilisation d'une cellule de levure selon l'une quelconque des revendications 9 à 11 pour
- la conversion et la métabolisation, en particulier la fermentation recombinante, d'un biomatériau contenant du L-arabinose,
- la production de produits chimiques ou de biocarburants biosourcés,
- la production de bioalcools, de préférence de bioéthanol et/ou de biobutanol.

13. Procédé de production d'un bioalcool comprenant les étapes suivantes :
(a) la conversion d'un milieu contenant une source de L-arabinose avec une cellule selon l'une quelconque des revendications 9 à 11, qui convertit le L-arabinose en bioalcool,
(b) facultativement l'obtention du bioalcool,
dans lequel le bioalcool est du bioéthanol et/ou du biobutanol.

14. Procédé de production d'un produit de métabolisation comprenant les étapes suivantes :
(a) la conversion d'un milieu contenant une source de L-arabinose avec une cellule selon la revendication 11, qui convertit le L-arabinose pour produire le produit de métabolisation,
(b) facultativement l'obtention du produit de métabolisation,
le produit de métabolisation étant de préférence choisi dans le groupe comprenant les produits chimiques ou les biocarburants biosourcés, tels que l'acide lactique, l'acide acétique, l'acide succinique, l'acide malique, le 1-butanol, l'isobutanol, le 2-butanol, d'autres alcools, les acides aminés, le 1,3-propanediol, l'éthylène, le glycérol, un antibiotique de type β-lactamine ou une céphalosporine, les alcanes, les terpènes, les isoprénoïdes ou la molécule précurseur amorphadiène du médicament antipaludique artémisinine.

15. Procédé selon la revendication 13 ou 14, dans lequel le milieu contient une source de carbone supplémentaire, en particulier un hexose, plus particulièrement le glucose.
